(19) Europäisches Patentamt
European Patent Office
Office européen des brevets

(11) EP 4 390 959 A1

(12) **EUROPEAN PATENT APPLICATION**

(43) Date of publication:
**26.06.2024 Bulletin 2024/26**

(21) Application number: **23217258.5**

(22) Date of filing: **15.12.2023**

(51) International Patent Classification (IPC):
***G16H 30/20*** *(2018.01)* ***G16H 30/40*** *(2018.01)*
***G16H 50/20*** *(2018.01)* *G06T 7/00 (2017.01)*

(52) Cooperative Patent Classification (CPC):
**G16H 30/40; G16H 30/20; G16H 50/20;**
G06T 7/0012; G16H 50/70

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **22.12.2022 US 202218087200**

(71) Applicant: **Optellum Limited**
**Oxford, Oxfordshire OX1 1BY (GB)**

(72) Inventors:
- **Heames, Brennen**
**Oxford, OX1 1BY (GB)**
- **Chometon, Quentin**
**Oxford, OX1 1BY (GB)**
- **Montilva, Carlos Federico Arteta**
**Oxford, OX1 1BY (GB)**
- **Kadir, Timor**
**Oxford, OX1 1BY (GB)**

(74) Representative: **Optimus Patents Limited**
**Peak Hill House**
**Steventon**
**Basingstoke, Hampshire RG25 3AZ (GB)**

# (54) SYSTEM AND METHOD FOR ANALYSING MEDICAL IMAGES

(57) A computer implemented method and device for analysing medical scan images from a single patient is described. The method comprising the steps of: receiving an input of a plurality of medical scan images for the single patient: providing an input of one or more characterised features from the medical scan images, in addition to the plurality of medical scan images to an optimisation model, where the optimisation model outputs the characterised features and medical scan images sorted according to a predefined relevance criteria; providing an output to a user, related to the medical scan images according to the result of the predefined relevance criteria, that comprises a workflow showing the order in which the plurality of scans should be reviewed. The device comprises one or more processors to execute the method steps.

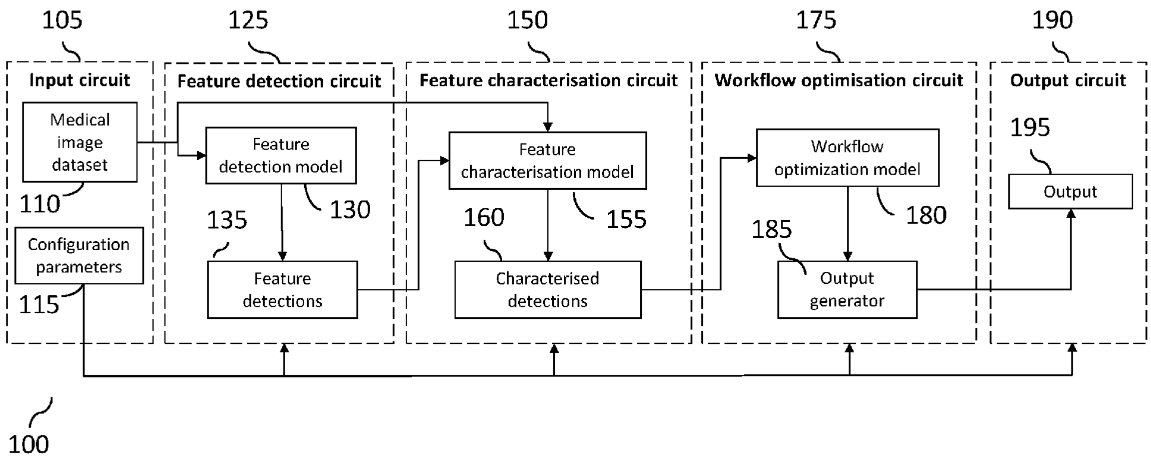


FIG. 1

EP 4 390 959 A1

## Description

### Field of Invention

This invention relates to the field of Computer Aided Diagnosis (CADx) devices and methods for assisting the interpretation of medical images to support clinicians in healthcare. In particular, the field relates to risk Computer Aided Diagnosis devices to assist the reading and reporting of medical images by radiologists and the interpretation of the radiologist's report by the physician responsible for patient care.

### Background of Invention

In the field of medical imaging, a variety of technologies can be used to investigate biological processes and anatomy. The following examples are types of scan that may be used to provide medical images: X-Ray; Computed Tomography (CT); Ultrasound (US); Magnetic Resonance Imaging (MRI); Single Photon Emission Tomography (SPECT); and Positron Emission Tomography (PET). Each type of scan is referred to as an "imaging modality".

Typically, a scan provides a "dataset". The dataset comprises digital information about the value of a variable at each of a plurality of spatial locations in either a two-dimensional or (more typically) a three-dimensional space. As a specific example, a CT scan may provide images of the chest of a patient. Such a CT scan might, as a more specific example, show lung nodules in the chest.

Computer Aided Detection (CADe) devices serve to assist clinicians in assessing the medical images. CADe devices need to provide a clinician with standardised, objective and repeatable information. The information typically relates to particular anatomical regions, including both normal tissue and lesions, within a person. CADe devices may be used as a so-called 'Second Reader' device. Second Reader Devices are based on an approach whereby a radiologist first looks at an image resulting from a scan, for example a mammogram. The radiologist will then, based on training and experience, identify areas of the scan where the radiologist considers that there may need to be a further investigation, for example a biopsy. However, the radiologist can then consider the CADe findings. Those findings might involve a display to highlight any additional suspicious regions on the mammogram. The radiologist will then, based on training and experience, look at those further areas of the scan. The CADe device is thereby performing a second look at the scan. The results of the second look at the scan may be that the radiologist will be directed to areas of the scan that he/she had overlooked. In this way, CADe devices are designed to reduce 'false negatives', which are also termed 'missed findings'. Thus, CADe devices perform a support role to clinicians. Examples of commercial CADe devices for lung health include 'Veolity Lung™' from MeVis, 'Veye Chest™' from Aidence, and 'Lung AI™' from Arterys.

Computer Aided Diagnosis (CADx) devices are a related technology to CADe. CADx devices attempt to solve a different problem and relate generally to risk assessment. Instead of focussing on potentially missed findings as in CADe, they try to assist the user to classify findings correctly, for instance, either as malignant or benign in the case of potentially cancerous lesions. They rely on the user to identify abnormalities, and typically provide a score that is indicative of the risk of malignancy. Examples of commercial CADx devices are the breast cancer screening product 'Transpara™' from 'Screenpoint™', and the lung cancer product Virtual Nodule Clinic (VNC) from Optellum. There are many non-clinical CADx devices in the academic literature.

In the space of lung cancer, CADx devices have traditionally focused on evaluating lung nodules to produce malignancy risk scores, with the aim of helping clinicians make more appropriate decisions for the patient. Examples of such lung cancer CADx devices include the VNC from Optellum, and qCT-Lung from qure.ai. The connection between risk assessment and clinical decision-making lies in that the most appropriate next step for a given patient with a lung nodule depends on the risk of it being malignant. For example, higher risk cases would warrant shorter term actions (e.g., PET/CT, biopsy, or short-term CT follow-up), while lower risk cases can be addressed with longer term CT follow-ups. The consensus of experts on how to link risk and clinical decision is collected in clinical guidelines published regularly by the relevant medical societies. In the case of lung cancer, these guidelines include those from the British Thoracic Society [1] and the Fleischner Society [2]. In these guidelines, risk assessment of the lung nodule is central to the decision-making process, hence it is thought that CADx devices that help clinicians better assess risk can lead to better management.

In addition to the risk assessment of nodules, the patient management process is critical in order to properly schedule follow-up action in line with nodule risk. The management of lung nodule patients is typically centralised to lung nodule clinics, where a team of thoracic medicine specialists (including radiologists, surgeons and pulmonologists) coordinate the optimal clinical decision for each patient. Lung nodule clinics in some cases use management software such as Optellum's VNC, which has been shown to reduce patient drop-out from nodule management and make the enrolment of new patients more efficient. Typically, a radiologist will make recommendations for patient follow-up based on findings in a new CT scan - for example if they identify a nodule with high risk of malignancy. These recommendations need to be passed on to the management team of the lung nodule clinic so that patients can be prioritised for follow-up actions. Recommendations are generally made in a textual report which may or may not be integrated in the lung nodule clinic management software. With the recent rollout of lung cancer screening programs in the US and worldwide, radiologists typically need to make many decisions regarding lung nodule findings. For this reason, providing more efficient workflows for radiologists has the potential to improve patient outcomes by ensuring the quality and consistency of decision making by radiologists. However, the same considerations regarding workflow efficiency are potentially relevant to many medical imaging workflows and are not limited to a specific imaging modality or pathology. Moreover, other co-morbidities will typically play a role in the recommendation of the best clinical action for a given patient.

In the space of CADe and CADx systems, 'end-to-end' detection and risk assessment systems for lung nodules (i.e., combined CADe/CADx) can be found in the literature. Recently presented systems such as [3, 4, 5] perform end-to-end lung cancer risk assessment on a patient level. These could be used to decide which patients require close attention by the radiologist and have been tailored for lung cancer screening programs. The deployment of such systems could be expected to significantly reduce the radiology workload, which is already extremely high in many hospital systems. Nevertheless, efforts to increase the uptake of lung cancer screening (e.g., in the USA), or the raise of lung cancer screening programs and general lung health checks worldwide will mean that the radiologists will continue to face ever increasing workloads. Therefore, further innovations targeting the efficiency of the radiology workflow will still be very much needed.

[1] Callister, M. E. J., et al. "British Thoracic Society guidelines for the investigation and management of pulmonary nodules: accredited by NICE." Thorax 70.Suppl 2 (2015): ii1-ii54.
[2] MacMahon, Heber, et al. "Guidelines for management of incidental pulmonary nodules detected on CT images: from the Fleischner Society 2017." Radiology 284.1 (2017): 228-243.
[3] Ardila, D., et al. "End-to-end lung cancer screening with three-dimensional deep learning on low-dose chest computed tomography." Nature Medicine (2019) 25:6, 954-961
[4] Kiraly, A. et al. "Method and system for improving cancer detection using deep learning." US Patent Application Publication No. US 2021/0225511 A1, Jul. 22, 2021
[5] Lyman et al. "Lung Screening Assessment System." US Patent No. US10896753B2, Jan. 19, 2021

## Summary of the invention

In a first embodiment of the invention there is provided a computer implemented method for analysing medical scan images from a single patient comprising the steps of: receiving an input of a plurality of medical scan images for the single patient: providing an input of one or more characterised features from the medical scan images, in addition to the plurality of medical scan images to an optimisation model, where the optimisation model outputs the features and images sorted according to a predefined relevance criteria; providing an output to a user, related to the medical images according to the result of the predefined relevance criteria, that comprises a workflow showing the order in which the plurality of scans should be reviewed.

Preferably, wherein the output is at least one of: a display of medical images sorted according to the relevance criteria, a report containing information about the medical images sorted according to the relevance criteria.

In a preferred embodiment of the invention, the method further comprising the steps of: detecting one or more features of the plurality of medical scan images; and characterising the one or more detected features from the plurality of medical scan images; before providing the input to the optimisation model.

Preferably, the optimisation model only outputs the N most relevant features according to the relevance criterion, where N is a configurable parameter of the method.

In a preferred embodiment of the invention, the plurality of medical scan images is one of: CT scan, PET scan, MRI scan or SPECT scan. Further preferably, the medical scan image is a 2D image or a 3D image.

In a further preferred embodiment of the invention, the one or more medical scan images are images showing all or part of a lung, and the detected features are one or more of: lung nodule, features indicating the presence of at least one of emphysema, fibrotic tissue, consolidation and scarring. Preferably, the detected features are characterised according to one or more of: a malignancy score, an invasiveness score, a feature size.

In an embodiment of the invention, detecting the features is done with a first machine learning model, and characterising the features is done with a second machine learning model. Preferably, the first and second machine learning model uses a neural network, a support vector machine or a random forest algorithm.

In a preferred embodiment of the invention, the optimization model uses a machine learning model such as a neural network.

Preferably, the feature detection model is a single model that detects multiple feature classes simultaneously. Alternatively, the feature detection model comprises a plurality of models, each model detecting a certain class of feature.

Further preferably, the characterization model characterises all features in all classes for simultaneous consideration by the optimization model.

According to an embodiment of the invention, there is provided a device for analysing medical scan images from a single patient comprising: one or more processors configured to: receive an input of a plurality of medical scan images for the single patient: provide an input of one or more characterised features from the plurality of medical scan images, in addition to the medical scan images to an optimisation model, where the optimisation model outputs the features and images sorted according to a predefined relevance criteria; providing an output related to the medical images to a user according to the result of the predefined relevance criteria, that comprises a workflow showing the order in which the plurality of scans should be reviewed.

Preferably, the plurality of medical scan images is one of: CT scan, PET scan, MRI scan or SPECT scan, and the one or more medical scan images are images showing all or part of a lung.

In a preferred embodiment of the invention, the one or more processors are further configured to detect multiple classes of features in the plurality of input medical scan images.

Further preferably, the one or more processors are further configured to characterise the one or more detected features from the plurality of medical scan image, before the detected features are provided to the optimisation model.

In a preferred embodiment of the invention, detecting the features is done with a first machine learning model, and characterising the features is done with a second machine learning model, and the optimisation model also uses a machine learning model.

Further preferably, the output is at least one of: a display of medical images sorted according to the relevance criteria, a report containing information about the medical images sorted according to the relevance criteria.

## Description of drawings

Figure 1: Schematic illustrating the workflow optimisation system;

Figure 2: Embodiment of the Feature Detection Circuit for lung nodule detection in thoracic CT scans;

Figure 3: Embodiment of the Feature Detection Circuit for detection of multiple features in thoracic CT scans;

Figure 4: Embodiment of the Feature Characterisation Circuit for characterisation of lung nodules;

Figure 5: Illustration of a suitable training protocol for the workflow optimisation model;

Figure 6: Illustration of a suitable training protocol for the workflow optimisation model;

Figure 7: Embodiment of the Workflow Optimisation Circuit based on thoracic CT detections with results presented as DICOM secondary captures;

Figure 8: Example of a DICOM secondary capture.

## Detailed description

Terms used
AIS: Adenocarcinoma In Situ
COPD: chronic obstructive pulmonary disease
CT: computerised tomography
DICOM: Digital Imaging and Communications in Medicine
IA: Invasive Adenocarcinoma
IPF: Idiopathic pulmonary fibrosis
MIA: Minimally Invasive Adenocarcinoma
PACS: picture archiving and communication system
Secondary capture: images converted from a non-DICOM format to a modality independent DICOM format

## Overview of the invention

This invention describes a radiology workflow optimisation method and system or device (100) to assist in the review of medical scan images for the reporting of features in medical image datasets and the issuing of recommendations, including clinical recommendations, the core of the day-to-day radiology workload. Preferably the method and device can be used in existing CADx or CADe devices. The system or device and method of this invention targets the optimization of a radiology workflow once the radiologist has started to read a medical image. In a first embodiment of the invention a computer implemented method for analysing medical scan images from a single patient is provided. The method comprising the steps of: receiving an input of a plurality of medical scan images for the single patient: providing an input of one or more characterised features from the medical scan images, in addition to the plurality of medical scan images to an optimisation model, where the optimisation model outputs the features and images sorted according to a predefined relevance criteria; providing an output to a user, related to the medical images according to the result of the predefined relevance criteria, that comprises a workflow showing the order in which the plurality of scans should be reviewed. In an embodiment of the invention, a device for carrying out the method is also provided. The device comprising: one or more processors configured to: receive an input of a plurality of medical scan images for the single patient: provide an input of one or more characterised features from the plurality of medical scan images, in addition to the medical scan images to an optimisation model, where the optimisation model outputs the features and images sorted according to a predefined relevance criteria; and providing an output related to the medical images to a user according to a result of the predefined relevance criteria, that comprises a workflow showing the order in which the plurality of scans should be reviewed.

The device includes one or more processors configured to control: an input circuit (105), a feature detection circuit (125), a feature characterisation circuit (150), a workflow optimisation circuit (175) and an output circuit (190). The input circuit (105) parses medical image datasets (110) as collected in hospital systems into a preferred format for analysis. The feature detection circuit (125) identifies occurrences of one or more type of feature in an input medical image dataset (110), preferably this identification is done automatically. The input medical image data set comprises a plurality of images from a single patient The feature characterisation circuit (150) classifies or scores each identified feature according to one or more pre-defined relevance criteria, again, this is preferably done automatically by the circuit. The workflow optimisation circuit (175) organizes the detected image features, considering their characterisation, according to at least one predefined relevance criteria, with the objective of producing a reading workflow for the radiologist to follow when reviewing the medical image for a single patient as to make more efficient use of their reading time. The output circuit (190) interfaces the workflow optimisation system with other hospital systems. In a preferred embodiment of the invention, the device for analysing medical scan images from a single patient comprises one or more processors configured to: receive an input of a plurality of medical scan images for the single patient: provide an input of one or more characterised features from the plurality of medical scan images, in addition to the medical scan images to an optimisation model, where the optimisation model outputs the features and images sorted according to a predefined relevance criteria; providing an output related to the medical images to a user according to the result of the predefined relevance criteria, that comprises a workflow showing the order in which the plurality of scans should be reviewed.

By detecting and characterizing clinically relevant features from the medical scan images, preferably automatically, then organizing the findings in a way that helps optimise a radiology workflow, and providing an output related to the medical images to a user according to the result of the predefined relevance criteria that comprises a workflow showing the order in which the plurality of scans should be reviewed, radiologists can spend less time in laborious but automatable tasks and shift focus to reviewing and curating results. As a consequence, radiologists can reduce the time invested in clinically trivial cases and prioritise those patients who require closer attention.

Figure 1 shows a schematic illustrating the workflow optimisation system or device, with the components used in the method of this invention. The system comprises an input circuit (105), that receives medical scan image dataset (110). Preferably, the medical image datasets comprises a plurality of medical scan images, where the medical scan images are one of: CT scan, PET scan, MRI scan or SPECT scan. Preferably, the medical scan image is a 2D image or a 3D image. In an embodiment of the invention, the one or more medical scan images are images showing all or part of a lung, and the detected features are one or more of: lung nodule, features indicating the presence of at least one of emphysema, fibrotic tissue, consolidation and scarring. In addition to parsing medical image datasets, the input circuit (105) may optionally be provided with configuration parameters (115) which are passed to all subsequent circuits in the system.

The input circuit then provides an input to a feature detection circuit (125). This circuit comprises a feature detection model (130), and feature detections (135). In some embodiments of the invention the feature detection model is a neural network. The output of the feature detection circuit is provided to a feature characterisation circuit (150), which comprises a feature characterisation model (155) that outputs a set of characterised detections (160). The output of the feature characterisation circuit (150) goes to the workflow optimisation circuit (175). This circuit comprises a workflow optimisation model (180), and an output generator (185). The results of the workflow optimisation circuit (175) pass to the output circuit (190), where the overall output is generated. These various components of the workflow optimisation will be described in more detail as this description progresses.

Feature detection circuit

The feature detection circuit (125) constitutes the first part of a preferred embodiment of the workflow optimisation system. It takes as input a medical image dataset (110) consisting of one or more medical images of the same patient, and outputs a list of detected features alongside their locations for any feature found in the images (135). The feature detection circuit can also take as input additional (non-image) configuration parameters (115), although some embodiments of the device do not require this input. An example of the usage of configuration parameters for the feature detection circuit could be to set a size threshold below which smaller detected features are discarded

Radiological medical image datasets comprise either 2D images or 3D image volumes.
In its general form, the output of the feature detection circuit indicates the location of any features of interest in the 2D or 3D image. In some examples, the location of features of interest in a CT volume are indicated with {x,y,z} coordinates in the coordinate system of the CT scanner. In other examples, locations could be represented by bounding boxes, spheres or segmentations

As illustrated in Figure 1, the medical image dataset (110) given as input to the feature detection circuit (125) is first passed to the feature detection model (130), for detection of one or more features of the plurality of medical scan images. The feature detection model (130) gives as output the locations within the image where a given type of feature is likely to be present. In some embodiments, the feature detection model also returns additional information associated with each identified feature, a typical example being the confidence of each detection. In the simplest case, the locations of interest from the feature detection model are given directly as the output of the feature detection circuit. The initial locations of interest may also be transformed in some way before being given as output, for example to remove duplicate detections or to apply a likelihood threshold in order to reduce the number of outputs. In other examples, the feature detection model (130) identifies the presence of more than one category of image feature, for example pulmonary nodules, emphysema, fibrotic tissue, consolidation and scarring. In the scenario where more than a single category of image features are considered, some embodiments of the feature detection model (130) will consist of a single model that detects multiple feature classes simultaneously, while in other examples the model is itself an ensemble of models that each detect a certain class of feature. Alternatively, the feature detection model comprises a plurality of models, each model detecting a certain class of feature. Further preferably, the characterization model characterises all features in all classes for simultaneous consideration by the optimization model.

The feature detection model (130) within the feature detection circuit is typically based on a machine learning model trained to detect the feature of interest, while the specific architecture may take a variety of forms. In some example implementations, the feature detection model (130) is based on a neural network. In other examples, the underlying machine learning architecture of the feature detector is a Support Vector Machine or a Random Forest algorithm.

Typically, the feature detection model (130) operates by computing the likelihood of feature presence at every position in the input image dataset and returning any locations where the likelihood of feature presence is above a threshold. In other examples, the feature detection model (130) computes the likelihood of feature presence in locations of interest in the input image dataset such as salient image locations. In some examples of the feature detection circuit (125), an operating threshold is supplied as an additional parameter (115), with any detected features below this threshold being discarded. In embodiments of the circuit that detect multiple classes of features, multiple operating thresholds may be supplied. The values of the operating thresholds are typically chosen according to the clinical problem: if the cost of missing an instance of a feature is high, the threshold can be set to a lower value to increase the sensitivity of the device. Equally, the threshold may be set to a high value in order to reduce number of false positive detections.

The feature detection circuit (125) outputs a list of findings identified in the medical image, which is passed to the feature characterisation circuit (150). Preferably, the feature characterisation circuit may characterise the one or more detected features from the plurality of medical scan image; before providing the input to the optimisation model.
In some examples, the feature detection circuit (125) returns only a maximum number of detections of each feature class in an image, specifiable as an additional parameter supplied to the circuit.

*Embodiment 1: Detection of lung nodules in thoracic CT scans.*

In this embodiment of the invention, the feature detection circuit (125) detects lung nodules in a medical image dataset, preferably, a thoracic CT scans. As shown in Figure 2, an image dataset, preferably a CT dataset is supplied as input to the feature detection circuit (125) in a suitable format e.g., DICOM (110). The feature detection circuit (125) handles conversion of the data via image dataset pre-processing (205) to an input format suitable for the feature detection model (130) such as a volumetric data array. In this embodiment of the invention, the feature detection model (130) is trained to detect the presence of features using a training dataset. In this embodiment of the invention, the dataset is comprised of many thoracic CT scans, and the detected features are lung nodules. Preferably, the feature detection model (130) is based on a neural network. The feature detection model (130) outputs the locations of one or more features, preferably, lung nodules identified in the image dataset, preferably along with other information such as their estimated diameter and the confidence of the detection. Subsequently, the feature detection circuit (125) computes a list of distinct lung nodule detections (135), with confidence scores above the supplied operating thresholds. The list of detections, each detection comprising the {x,y,z} coordinates, diameter, and confidence score, are returned as the final output of the feature detection circuit (125).

*Embodiment 2: Simultaneous detection of multiple features in thoracic CT scans.*

An embodiment of the feature detection circuit (125) that detects multiple feature classes is shown in Figure 3. Preferrable the circuit comprises one or more processors that is further configured to detect multiple classes of features in the plurality of input medical scan images. This example embodiment of the invention is capable of detecting lung nodules, emphysema, fibrotic tissue, consolidation and scarring in medical scan datasets; preferably the scan datasets are thoracic CT scans. Following image dataset pre-processing (205), the image is fed to a feature detection model (130) which has preferably been trained to detect the relevant categories of feature. In this embodiment, the feature detection model (130) is a neural network which simultaneously detects features of each of the categories of interest. The detections are processed (210) based on one or more additional parameters configured according to the clinical problem. Finally, detections for all the different feature categories form the output of the feature detection circuit (135).

Feature characterisation circuit

Results of the feature detection circuit (135), for all the different embodiments of the invention are passed to the feature characterisation circuit (150) along with the medical image dataset (110), for further analysis and processing. Like the feature detection circuit (125), the feature characterisation circuit (150) is preferably based on a prediction model that has been built using machine learning algorithm, the feature characterisation model (155). The feature characterisation model (155) is in this case trained to predict characteristics of each of the features detected by the feature detection circuit (125), which in some examples consist of different categories of features.
Analogous to the feature detection circuit (125), the predictive model of the feature characterisation circuit may itself be an ensemble of models specialised to characterise different categories of feature, or different characteristics of single feature category. A characteristic is here defined as a property with a numerical value, or a categorisation, that is associated with a detected instance of a feature. For the purposes of the feature characterisation circuit (150), it should be possible to determine or predict the characteristic from the image. In many examples, predictions of characteristics are represented as probabilities.
In general, the feature characterisation circuit (150) extracts one or more image crops or sub-volumes from the 2D image or 3D image volume, respectively. The extracted regions from the image or image volume are typically centred on each of the detected features. For each detected feature supplied as input to the feature characterisation circuit (150), the feature characterisation model (155) outputs a score or categorisation given the appearance of the detected feature on the image. In some cases, multiple detections of a given category of features may be present in the same image crop or sub-volume. Here, the feature characterisation model (155) typically only outputs a single score or categorisation which considers the presence of all detected features.
The feature characterisation model (155) may take a variety of forms including neural networks and other machine learning models such as Support Vector Machines or Random Forests.
As illustrated in Figure 1, the output of the feature characterisation circuit extends the list of detected features (135) with feature characterisation information. That is, one or more lists of detected features, each corresponding to a certain category of image feature (160), now contains the locations of the set of detected features along with characterisation information produced by the feature characterisation circuit. This output is provided to the workflow optimisation circuit (175).
Various embodiments of the feature characterisation circuit (150) are described below. All embodiments describe the characterisation of features in a medical scan dataset. Preferably, the features are lung nodules detected in thoracic CT scans by e.g., *Embodiment 1* of the feature detection circuit (125). It would be clear to a person skilled in the area how the feature characterisation circuit would be applicable to conditions other than lung nodules if they can be diagnosed from medical images.

*Embodiment 1: Characterisation of lung cancer risk.*

In an embodiment of the invention features detected by the feature detection circuit (125) are characterised by the feature characterisation circuit (150) according to a particular criterion. In a preferred embodiment of the invention, the features are lung nodules that are characterised according to their malignancy risk. This embodiment is appropriate e.g., for a use case of the workflow optimisation system (100) specialised for thoracic CT scans; for instance, optimising the workflow of radiologists carrying out imaging as part of a lung cancer screening program. Assessment of the risk of identified lung nodules being malignant can be time consuming and can vary widely according to the experience and expertise of the radiologist, among other subjective factors. Automated risk assessment can therefore make the radiology workflow more accurate and efficient by presenting the highest risk findings to the radiologist first.

This embodiment of the feature characterisation circuit (150) for lung cancer risk as shown in Figure 4, contains a feature characterisation model (155) which preferably takes as input a sub-volume extracted from the whole CT scan. Accordingly, the feature characterisation circuit (150) first extracts scan image sub-volumes centred on the locations given as input to the circuit in a pre-processing step (405). The image dataset is preferably a set of thoracic scan images, and CT sub-volumes are fed successively to the feature characterisation model, and each CT sub volume is assigned a malignancy score, for example, as a probability in the range 0-1. Finally, the feature characterisation circuit (150) outputs the same set of lung nodule detections, now annotated with their lung cancer risk (160).

For this task the feature characterisation model (155) is trained on a dataset of thoracic CT scans, some of which containing lung nodules annotated as either benign or malignant.

*Embodiment 2: Characterisation of lesion invasiveness.*

In another embodiment of the feature characterisation circuit (150), lung nodules on a medical scan image are characterised by their potential invasiveness, assuming they were malignant. Predicted cancer invasiveness is an important factor to consider when deciding how to prioritise the reporting of lung nodules in patients with multiple detected nodules as it affects the urgency with which the patient should be followed up.

As in the previous embodiment, training the feature characterisation circuit (150) to estimate the level of invasiveness of a lung nodule assuming it is a cancer can be done from a dataset of images of lung nodules annotated with information relevant to invasiveness. In some examples, the invasiveness information comes in the form of the histological type of the cancer. For instance, in the case of lung cancer, Adenocarcinoma In Situ (AIS), Minimally Invasive Adenocarcinoma (MIA) and Invasive Adenocarcinoma (IA) represent different levels of invasiveness of a similar type of lung cancer as determined by their histological characterisation.

As in the previous embodiment, the feature characterisation model (155) also operates on sub-volumes of a thoracic CT scan provided as input (110), centred on the lung nodule detections also given as input (135). Here, the circuit outputs a list of lung nodule detections now annotated with invasiveness scores (160).

*Embodiment 3: Characterisation of both lung cancer risk and lesion invasiveness.*

In a further embodiment of the feature characterisation circuit (150), multiple characteristics are computed for a single category of feature. Specifically, both lung cancer risk and lesion invasiveness are computed for each detected lung nodule in a thoracic CT scan. As illustrated in Figure 4, the output of this embodiment is a list of properties corresponding to each detected nodule (160).

The feature characterisation model (155) in this case consists of a single neural network trained to carry out multiple tasks i.e., prediction of both lung cancer risk and lung cancer invasiveness.

Workflow optimisation circuit

Referring back to Figure 1, the workflow optimisation circuit (175) takes as the input a set of one or more characterised detections (160) provided by the feature characterisation circuit (150), and then organises the characterised detections in a way that eases the workflow of the radiologist, and generates an output with the results in an appropriate format as described through various embodiments below. Specifically, this workflow optimisation circuit (175) transforms the output of the detection circuit (125) and characterization (150) circuits into an organized workflow for the radiologist to follow when reviewing the medical image in a way that they make a more efficient use of the reading time.

Depending on the imaging modality, imaging protocol, and image features of interest, the set of findings in an image dataset is presented with the most clinically relevant findings displayed first. In this way, a radiologist using the system is not always required to review all system findings e.g., in the case that the most relevant finding determines the clinical recommendation issued by the radiologist, or can prioritise the review of certain findings over others that are less clinically relevant.

In an embodiment of the invention, input is provided to the workflow optimisation circuit and the top N most clinically relevant features are output by the output generator, where N is configurable by the user.

*Workflow optimisation model*

The central element of the workflow optimisation circuit (175) is a workflow optimisation model (180), which carries out the logic of the circuit (175). It takes as input the list of characterised detections (160) and any required configuration parameters (115) which are set according to the clinical setting. In a preferred embodiment of the invention the input to the optimisation model further comprises information on the location of the detected features.
The main output of the optimisation model is a list of the detected features, of all relevant categories, sorted according to their clinical relevance. Preferably, the output is at least one of: a display of medical images sorted according to the relevance criteria, a report containing information about the medical images sorted according to the relevance criteria. In some examples of the workflow optimisation circuit, the optimisation model also outputs a clinical recommendation guidance e.g., recommendations for the patient's clinical pathway. Recommendation guidance is typically based on the corresponding clinical guidelines applicable to each of the categories of features detected. In a preferred embodiment of the invention a clinical recommendation is generated for at least the most clinically relevant feature in at least one of the feature categories.

The workflow optimisation model (180) may take a variety of forms depending on the embodiment of the invention. For example, the logic of the workflow optimisation model may be rule based, or use a more complex computation such as a machine learning model. These are illustrated in the following example embodiments:

*Embodiment 1 of workflow optimisation model (180): Single feature category with single characterisation.*

In this embodiment of the workflow optimisation model (180), only a single type of feature category and with a single characterisation is given as input, for instance lung nodules with associated risk of malignancy. The list of detected lung nodules is then sorted by their associated malignancy risk. Other examples of appropriate feature characterisations in this case include: regions of pulmonary fibrosis, characterised by the specific type of fibrosis; regions of emphysema, characterised by the severity of the emphysema. In some versions of the embodiments, configuration parameters (115) supplied to the circuit are used to refine the output of the workflow optimisation model e.g., based on thresholds for minimum diameter of the nodule, so as to exclude the smallest detected nodules which are not associated with clinical risk.

In addition to the sorted list of nodule detections according to malignancy risk, in this embodiment the workflow optimisation model provides a patient-wise clinical recommendation considering all detected and characterised nodules. For example, the appropriate follow-up for the nodule with the highest risk of malignancy, according to the relevant clinical guidelines.

*Embodiment 2 of workflow optimisation model (180): Single category of features with multiple characterisations.*

In this embodiment of the workflow optimisation model (180), multiple feature characterisations are provided as input, all belonging to a single category of feature. The workflow optimisation model (180) then computes an ordering of the findings by considering all feature characterisations simultaneously. For example, lung nodules are detected by the feature detection circuit (125), and risk of malignancy and predicted invasiveness (e.g., according to their histological type) of a potential malignant nodule are given as characterisation of each lung nodule by the feature characterisation circuit (150).

In an example of this embodiment, where the nodule invasiveness potential is codified as a probability of invasiveness within a pre-specified time period t, the clinical relevance of each of the lung nodule findings can be computed as:

$$lung_nodule_relevance = p(malignancy|im) * p(invasiveness|im, t) \quad (1)$$

That is, the relevance of each lung nodule finding is scored as the probability of malignancy, as estimated form the medical image, multiplied by the probability of the lung nodule invading surrounding tissue within as time period t.

In other example embodiments of the lung nodule characterised by malignancy risk and invasiveness, where the invasiveness is extracted from the histological type predicted from the image, the relevance of the detected nodules is computed as:

$$lung_nodule_relevance = p(malignancy|im) * p(invasive_type|im) \quad (2)$$

Here, the relevance of each lung nodule finding is scored as the probability of malignancy, as computed from the medical image, multiplied by the probability of the lung nodule being of a histological type considered invasive (e.g., Invasive Adenocarcinoma) as opposed to not a non-invasive type (e.g., Adenocarcinoma in Situ).

*Embodiment 3 of workflow optimisation model (180): Multiple categories of features with multiple characterisations.*

In this embodiment of the workflow optimisation model (180) multiple feature characterisations are provided as input for each of multiple categories of feature. Therefore, the logic behind the organisation of findings may be more complex. As an example of this embodiment, the feature detection circuit (125) detects different features, such as lung nodules as well as pulmonary fibrosis and lung emphysema. The feature characterisation circuit (150) then characterises the feature for a specific criterion, for example: the risk of malignancy for the lung nodules, type of fibrosis, and severity of emphysema. In this example embodiment, the relevance of lung nodules is determined, as before, from their malignancy risk. However, the workflow optimisation circuit (175) needs to prioritise over the categories of findings, that is, between the reporting of the fibrosis, lung nodules, and emphysematous lung tissue. In this example embodiment of the workflow optimisation model, the emphysema finding may take the lowest priority over the remaining categories, while the top category is decided between the fibrosis and lung nodules such that the fibrosis category is prioritised over the lung nodule category if the probability of a concerning type of fibrosis, e.g., IPF, is higher than the highest probability of malignancy:

$$top_category = \begin{cases} Nodules, if\ argmax_n\ (P_n(malignancy|im))\ \forall\ n\ \in N > \ P(IPF|im) \\ \qquad Fibrosis, otherwise \end{cases} \quad (3)$$

Here, N is the list of detected lung nodules.

In another example of this embodiment where there is a more extensive list of categories and characterisations for each category, the organisation model is based on a machine learning model such as a neural network trained to rank the clinical relevance of the various characterised findings.

The training protocol for some examples of this model is shown in Figure 5. As shown, training data is loaded at (505). This is provided to the workflow optimisation model (150), and then feature rank predictions (510) are made. A prediction error (520) is computed from the feature rank predictions using a ground truth feature ranking (515). The prediction error (520) is then used to update the parameters of the workflow optimisation model (525), and the final step is a test for training convergence (530). If convergence is reached the training ends, but if not, the method returns back to step 505, and the steps are repeated. Key to enabling this training process is a dataset of characterised feature detections (of the form given as output by the feature characterisation circuit (150) annotated with ground truth labels, in this case consisting of feature-level rankings assigned by a radiologist (515).

Preferably, the training dataset is constructed by collecting historic clinical recommendations from a plurality of radiology reports. This required that a ranking of the clinical relevance of each finding can be discerned from each report. In some examples of the training method, additional training samples are gathered by sampling synthetic sets of characterised feature detections from an appropriate prior distribution and asking radiologists to annotate the ranking of the findings according to their clinical relevance.

In this embodiment of the workflow optimisation model, a patient-wise clinical recommendation is included in the output (195). Here, the workflow optimisation model (180) is also trained to issue a patient-wise clinical recommendation form the same set of characterised feature detections of the form (160). This task is non-trivial given the nonlinear dependence of pathologies on certain features, making a machine learning model trained for this task the most appropriate way to provide recommendation guidance to the radiologist using the system.

In this embodiment of the invention the workflow optimisation model determines, preferably automatically the clinical relevance of an image finding relative to other classes of image findings based, where each image finding is associated to at least one characterisation

An alternative example training protocol is shown in Figure 6, following the same iterative process as described in (500). As shown, training data is loaded at (505). This is provided to workflow optimisation model (150), and then clinical recommendation predictions (610) are made. A prediction error (620) is computed from the feature rank predictions using a ground truth clinical recommendation (615). The prediction error (620) is then used to update the parameters of the workflow optimisation model (625), and the final step is a test for training convergence (530). If convergence is reached the training ends, but if not, the method returns to step 505, and the steps are repeated. For this task, the same training samples are used as for the feature ranking task (505). The major difference is that prediction error is computed based on ground truth clinical recommendations (615). This additional set of labels for the same training data should be gathered in the same way as before (e.g., inferred from historic radiology reports).

*Output generator*

The final element of the workflow optimisation circuit (175) of Figure 1 is the output generator (185) which generates several possible outputs formats for the information generated by the workflow optimisation model (180).

The various embodiments of the workflow optimisation model (180) and the output generator (185) are not mutually exclusive; thus, some embodiments of the workflow optimisation circuit (175) are constructed from different embodiments of the building blocks. As an example, an embodiment of the workflow optimisation circuit (175) is illustrated in Figure 7. This embodiment is appropriate for a device specialised for thoracic CT scans, taking as input a list of characterised lung nodules (160) each with associated parameters, such as malignancy and invasiveness scores. In this embodiment, the workflow optimisation model (180) consists of a neural network, and the output generator (185) outputs the results as a stack of DICOM secondary captures. Preferably, the output generator produces a DICOM secondary capture, compatible with common image viewers for radiology, where the DICOM secondary capture series is organized following the feature ordering produced by the workflow optimisation mode.

Embodiments of the output generator are further described next.

*Embodiment 1 of output generator (185): DICOM secondary capture*

In this embodiment of the output generator (185), the results from the workflow optimisation model (180) are preferably presented to a user in the form of a DICOM secondary capture stack, as illustrated in Figure 8. The advantage of this embodiment is that the output can be visualized in any DICOM viewer commonly used by radiologists. The implication is that the radiologist is not required to break their workflow to use the device e.g., by switching to a separate workstation.

In an example of this embodiment, the detected features preferably correspond to lung nodules, and they are characterised by a parameter such as risk of malignancy. Each characterised feature reported by the feature characterisation circuit (150) is saved as a single DICOM file of secondary capture type, containing a snapshot of the finding along with risk of malignancy characterisation. This (non-image) information may be saved as metadata associated with the DICOM file, or displayed as an overlay on the snapshot (810) alongside any relevant metadata associated with the patient and the medical image dataset (805).

If multiple findings are reported in a single image dataset, the individual DICOM files associated to each finding are saved as part of the same DICOM series, along with their associated metadata modified to ensure they are loaded in the DICOM viewer according to ordering defined by the workflow optimisation model (180). In this way, a radiologist can quickly see the most relevant findings first, and in some cases, may be able to make a clinical decision without needing to review all automatic findings in the results. Multiple findings are in this case referenced on each individual secondary capture, allowing for a quick overview of all findings (820).

Additional information can be integrated into the DICOM secondary capture to assist the radiologist's workflow. In a further example of this embodiment, each detected feature is labelled and/or outlined in the secondary capture images. Finally, in an example of the workflow optimisation model (180) where diagnostic guidance is also produced, it can also be displayed on the secondary capture, or all secondary captures in the stack if multiple feature detections are reported. For example, information to guide interpretation of a malignancy risk score can be overlayed (815).

*Embodiment 2 of output generator (185): Pre-filling of results into a structured report.*

Having reviewed a medical image dataset a radiologist will typically submit their findings to a reporting system in the form of a report. Preferably, the output generator is configured to fill structured reports following the feature ordering produced by the workflow optimisation model. In this embodiment of the output generator (185), results from the workflow optimisation model (180) are pre-filled into a structured report which can be reviewed and edited by the radiologist before submitting to the reporting system. Pre-filling of results allows users to review any findings that will be reported, remove findings according to their own assessment of the image dataset, e.g., by removing findings that are not clinically relevant such as false positives of the feature detection circuit (125), add findings such as the false negatives of the feature detection circuit (125), and add any other information needed to complete the report. In this way, a major component of the cross-checking and summarising of results is automated, making the radiologist's workflow faster and less labour intensive, and reducing the chances of clinical errors.

In some examples of this embodiment, the report includes all findings in the medical image dataset. In other examples, only the most relevant findings are pre-filled into the report according to the configuration of the workflow optimisation circuit (175).

In a preferred embodiment of the invention, the workflow optimisation circuit, consists of a workflow optimisation model and an output generator, which receives a list of at least one category of features found in a medical scan image. Preferably, the output generator also receives at least one clinical characterisation of each of the medical scan images, and orders the features in the medical scan image in according to their clinical relevance.

A preferred embodiment of the invention comprises a combination of a feature detector circuit, feature characterisation circuit and workflow optimisation circuit.

Preferably, the workflow optimisation model determines the clinical relevance of a type of image finding based on more than one feature characterisation (i.e. embodiment 2 of the workflow optimisation model). Preferably, this determination is performed automatically.

This invention targets the optimization of a radiology workflow once the radiologist has started to read a medical image. This contrasts with the rather common systems that help decide which medical images should be reviewed first or even reviewed at all. The innovation mainly builds on top of systems that simultaneously detect lesions and assess their risk of disease from medical images. In particular, this invention adds the workflow optimization circuit which transforms the output of the detection and characterization circuits into an organized workflow for the radiologist to follow when reviewing the medical image in a way that they make a more efficient use of the reading time.

The invention is suitable for a system that can be installed in the radiology department of a hospital, clinic, healthcare facility or dedicated radiology or tele-radiology service, in help radiologist increase their efficiency when processing long backlogs of medical images. This can be, for instance, a software product that is installed directly in the radiology workstations, or delivered through standard radiology software such as PACS viewers.

The present invention has been described with reference to the accompanying drawings. However, it will be appreciated that the present invention is not limited to the specific examples herein described and as illustrated in the accompanying drawings. Furthermore, because the illustrated embodiments of the present invention may for the most part, be implemented using electronic components and circuits known to those skilled in the art, details will not be explained in any greater extent than that considered necessary as illustrated above, for the understanding and appreciation of the underlying concepts of the present invention and in order not to obfuscate or distract from the teachings of the present invention.

The invention may be implemented in a computer program for running on a computer system, at least including code portions for performing steps of a method according to the invention when run on a programmable apparatus, such as a computer system or enabling a programmable apparatus to perform functions of a device or system according to the invention.

A computer program is a list of instructions such as a particular application program and/or an operating system. The computer program may for instance include one or more of: a subroutine, a function, a procedure, an object method, an object implementation, an executable application, an applet, a servlet, a source code, an object code, a shared library/dynamic load library and/or other sequence of instructions designed for execution on a computer system. Therefore, some examples describe a non-transitory computer program product having executable program code stored therein for automated contouring of cone-beam CT images.

The computer program may be stored internally on a tangible and non-transitory computer readable storage medium or transmitted to the computer system via a computer readable transmission medium. All or some of the computer program may be provided on computer readable media permanently, removably or remotely coupled to an information processing system. The tangible and non-transitory computer readable media may include, for example and without limitation, any number of the following: magnetic storage media including disk and tape storage media; optical storage media such as compact disk media (e.g., CD ROM, CD R, etc.) and digital video disk storage media; non-volatile memory storage media including semiconductor-based memory units such as FLASH memory, EEPROM, EPROM, ROM; ferromagnetic digital memories; MRAM; volatile storage media including registers, buffers or caches, main memory, RAM, etc.

A computer process typically includes an executing (running) program or portion of a program, current program values and state information, and the resources used by the operating system to manage the execution of the process. An operating system (OS) is the software that manages the sharing of the resources of a computer and provides programmers with an interface used to access those resources. An operating system processes system data and user input, and responds by allocating and managing tasks and internal system resources as a service to users and programs of the system.

The computer system may for instance include at least one processing unit, associated memory and a number of input/output (I/O) devices. When executing the computer program, the computer system processes information according to the computer program and produces resultant output information via I/O devices.

In the foregoing specification, the invention has been described with reference to specific examples of embodiments of the invention. It will, however, be evident that various modifications and changes may be made therein without departing from the scope of the invention as set forth in the appended claims and that the claims are not limited to the specific examples described above.

Those skilled in the art will recognize that the boundaries between logic blocks are merely illustrative and that alternative embodiments may merge logic blocks or circuit elements or impose an alternate decomposition of functionality upon various logic blocks or circuit elements. Thus, it is to be understood that the architectures depicted herein are merely exemplary, and that in fact many other architectures can be implemented which achieve the same functionality.

Any arrangement of components to achieve the same functionality is effectively 'associated' such that the desired functionality is achieved. Hence, any two components herein combined to achieve a particular functionality can be seen as 'associated with' each other such that the desired functionality is achieved, irrespective of architectures or intermediary components. Likewise, any two components so associated can also be viewed as being 'operably connected, ' or 'operably coupled,' to each other to achieve the desired functionality.

Furthermore, those skilled in the art will recognize that boundaries between the above described operations merely illustrative. The multiple operations may be combined into a single operation, a single operation may be distributed in additional operations and operations may be executed at least partially overlapping in time. Moreover, alternative embodiments may include multiple instances of a particular operation, and the order of operations may be altered in various other embodiments.

However, other modifications, variations and alternatives are also possible. The specifications and drawings are, accordingly, to be regarded in an illustrative rather than in a restrictive sense.

In the claims, any reference signs placed between parentheses shall not be construed as limiting the claim. The word 'comprising' does not exclude the presence of other elements or steps then those listed in a claim. Furthermore, the terms 'a' or 'an,' as used herein, are defined as one or more than one. Also, the use of introductory phrases such as 'at least one' and 'one or more' in the claims should not be construed to imply that the introduction of another claim element by the indefinite articles 'a' or 'an' limits any particular claim containing such introduced claim element to inventions containing only one such element, even when the same claim includes the introductory phrases 'one or more' or 'at least one' and indefinite articles such as 'a' or 'an.' The same holds true for the use of definite articles. Unless stated otherwise, terms such as 'first' and 'second' are used to arbitrarily distinguish between the elements such terms describe. Thus, these terms are not necessarily intended to indicate temporal or other prioritization of such elements. The mere fact that certain measures are recited in mutually different claims does not indicate that a combination of these measures cannot be used to advantage.

## Claims

1. A computer implemented method for analysing medical scan images from a single patient comprising the steps of: receiving an input of a plurality of medical scan images for the single patient:

   providing an input of one or more characterised features from the medical scan images, in addition to the plurality of medical scan images to an optimisation model, where the optimisation model outputs the characterised features and medical scan images sorted according to a predefined relevance criteria; and
   providing an output to a user, related to the medical scan images according to the result of the predefined relevance criteria, that comprises a workflow showing the order in which the plurality of scans images should be reviewed.

2. A method as claimed in claim 1, wherein the output is at least one of: a display of medical images sorted according to the predefined relevance criteria, a report containing information about the medical images sorted according to the predefined relevance criteria.

3. A method as claimed in claim 1, further comprising the steps of:

   detecting one or more features of the plurality of medical scan images; and
   characterising the one or more detected features from the plurality of medical scan images; before providing the input to the optimisation model.

4. A method as claimed in claim 3, wherein the optimisation model outputs the N most relevant features according to the predefined relevance criteria, where N is a configurable parameter of the method.

5. A method according to claim 1, wherein the plurality of medical scan images is one of: CT scan, PET scan, MRI scan, SPECT scan.

6. A method according to claim 5, wherein the one or more medical scan images are images showing all or part of a lung, and the characterised features are one or more of: lung nodule, features indicating the presence of at least one of emphysema, fibrotic tissue, consolidation and scarring.

7. A method as claimed in claim 3, wherein the detected features are characterised according to one or more of: a malignancy score, an invasiveness score, a feature size.

8. A method as claimed in claim 3, wherein detecting the features is done with a first machine learning model, and characterising the features is done with a second machine learning model.

**9.** A method according to claim 8, wherein the first and second machine learning model uses a neural network, a support vector machine or a random forest algorithm.

**10.** A method as claimed in claim 8, wherein the feature detection model is a single model that detects multiple feature classes simultaneously.

**11.** A method as claimed in claim 8, wherein the feature detection model comprises a plurality of models, each model detecting a certain class of feature.

**12.** A method as claimed in claim 10, wherein the model for characterising the features characterises all features in all classes for simultaneous consideration by the optimization model.

**13.** A device for analysing medical scan images from a single patient comprising:
one or more processors configured to:
receive an input of a plurality of medical scan images for the single patient:

provide an input of one or more characterised features from the plurality of medical scan images, in addition to the medical scan images to an optimisation model, where the optimisation model outputs the features and images sorted according to a predefined relevance criteria; and
providing an output related to the medical images to a user according to a result of the predefined relevance criteria, that comprises a workflow showing the order in which the plurality of scan images should be reviewed.

**14.** The device of claim 13, wherein the one or more processors are further configured to detect multiple classes of features in the plurality of input medical scan images.

**15.** The device of claim 14, wherein the one or more processors are further configured to characterise the one or more detected features from the plurality of medical scan image, before the detected features are provided to the optimisation model.

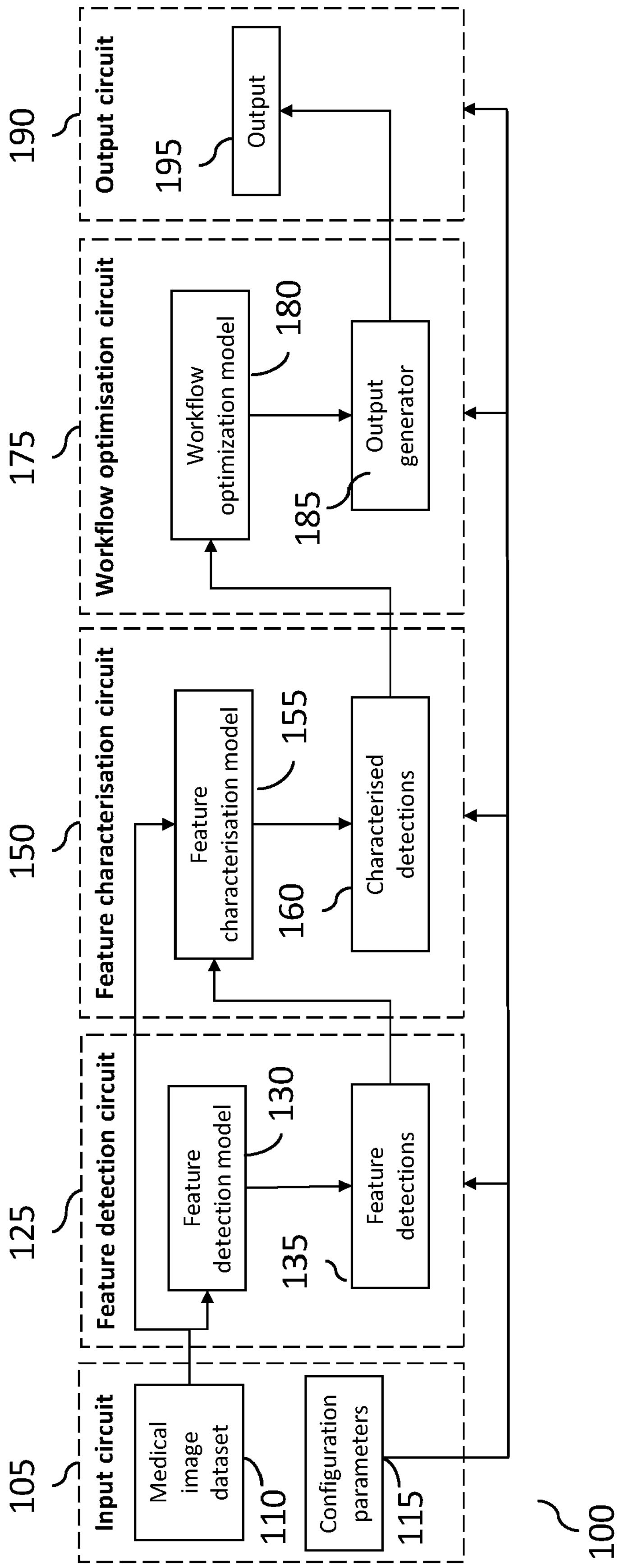
105
Input circuit
Medical image dataset
110
Configuration parameters
115
125
Feature detection circuit
Feature detection model
130
Feature detections
135
150
Feature characterisation circuit
Feature characterisation model
155
Characterised detections
160
175
Workflow optimisation circuit
Workflow optimization model
180
Output generator
185
190
Output circuit
Output
195
100

FIG. 1

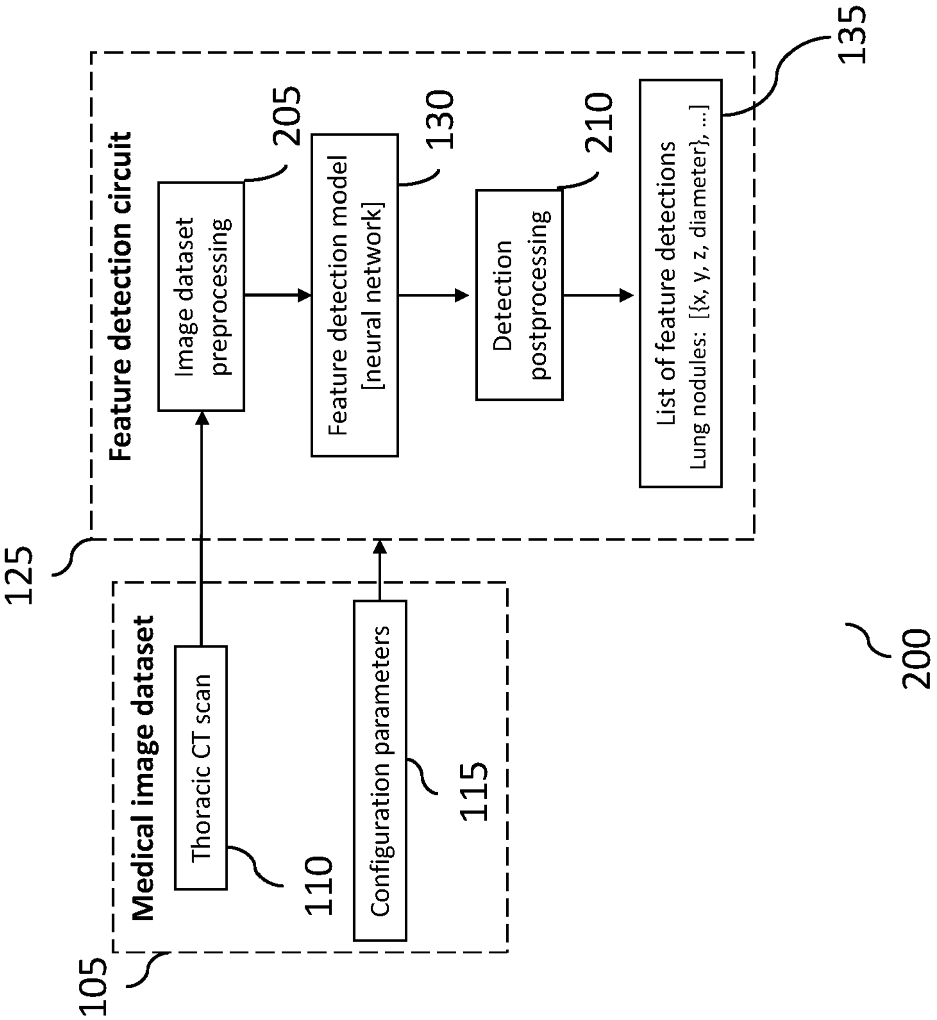
Medical image dataset
Thoracic CT scan
Configuration parameters
Feature detection circuit
Image dataset preprocessing
Feature detection model [neural network]
Detection postprocessing
List of feature detections
Lung nodules: [{x, y, z, diameter}, ...]
105
110
115
125
130
135
200
205
210

FIG. 2

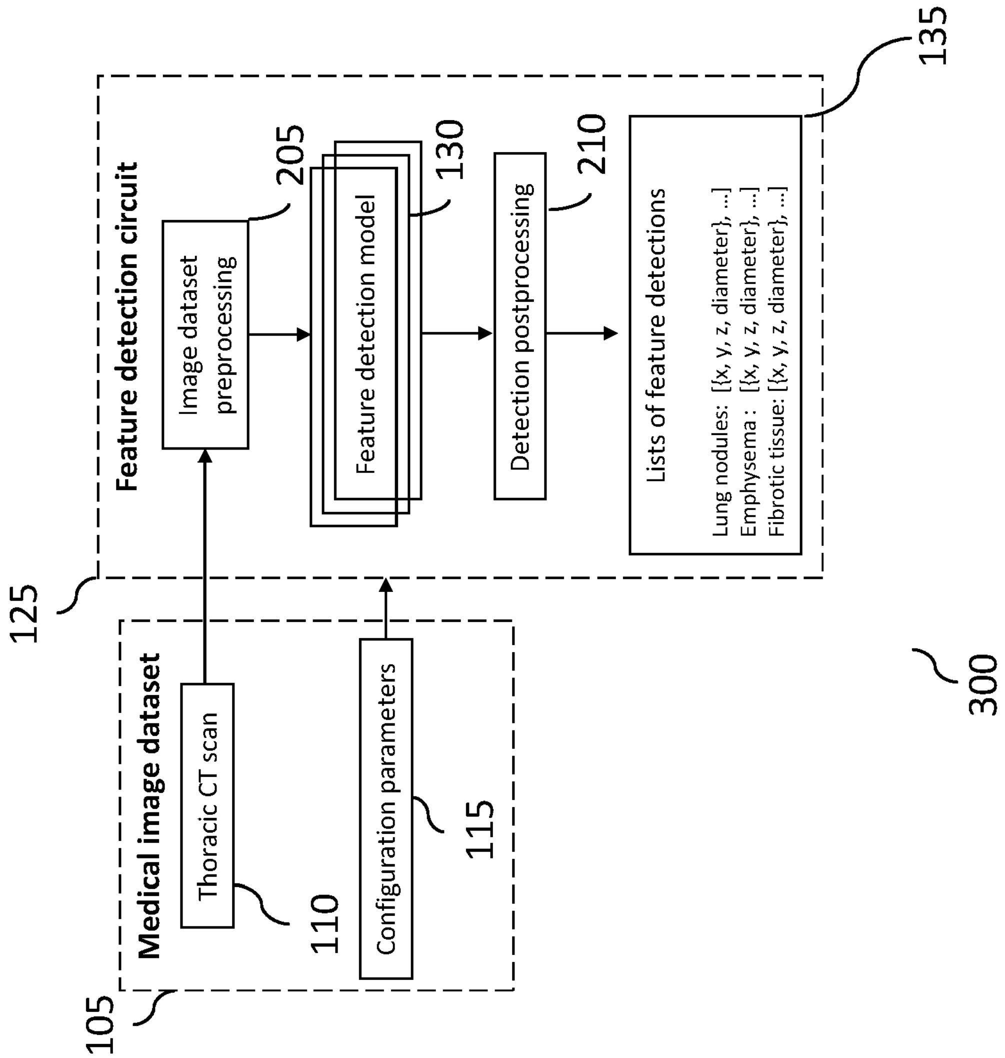
Medical image dataset
Thoracic CT scan
Configuration parameters
105
110
115
125
Feature detection circuit
Image dataset preprocessing
205
Feature detection model
130
Detection postprocessing
210
Lists of feature detections
Lung nodules: [{x, y, z, diameter}, ...]
Emphysema : [{x, y, z, diameter}, ...]
Fibrotic tissue: [{x, y, z, diameter}, ...]
135
300

FIG. 3

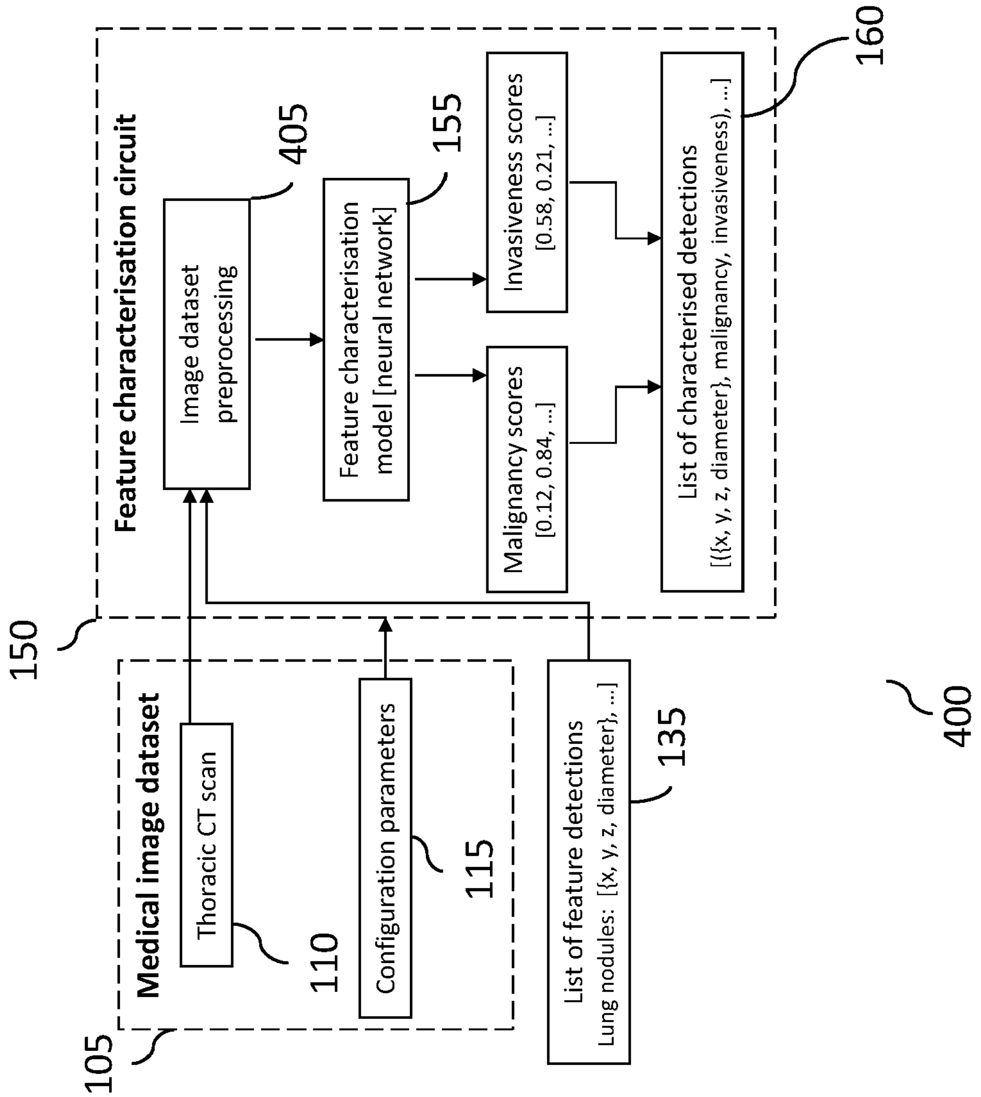
Feature characterisation circuit
Image dataset preprocessing
405
Feature characterisation model [neural network]
155
Invasiveness scores
[0.58, 0.21, ...]
Malignancy scores
[0.12, 0.84, ...]
List of characterised detections
[({x, y, z, diameter}, malignancy, invasiveness), ...]
160
150
Medical image dataset
Thoracic CT scan
110
Configuration parameters
115
105
List of feature detections
Lung nodules: [{x, y, z, diameter}, ...]
135
400

FIG. 4

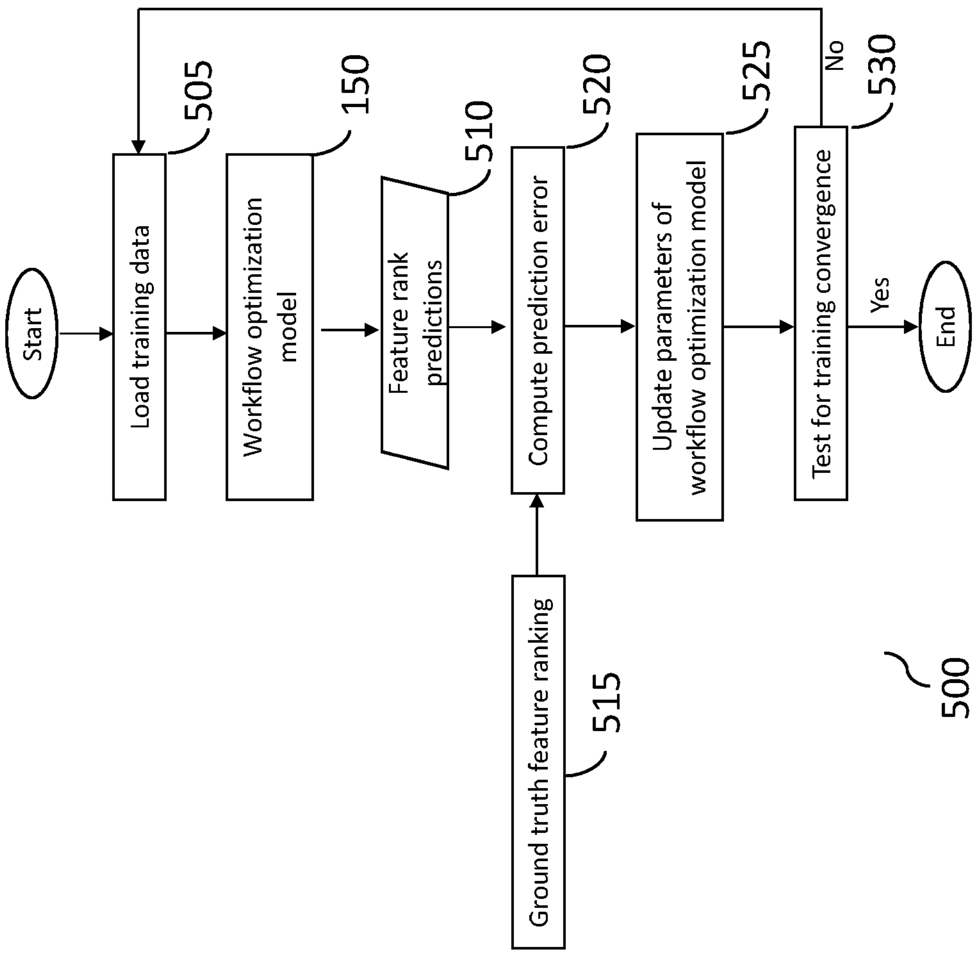
Start
Load training data
505
Workflow optimization model
150
Feature rank predictions
510
Compute prediction error
520
Ground truth feature ranking
515
Update parameters of workflow optimization model
525
Test for training convergence
530
No
Yes
End
500

FIG. 5

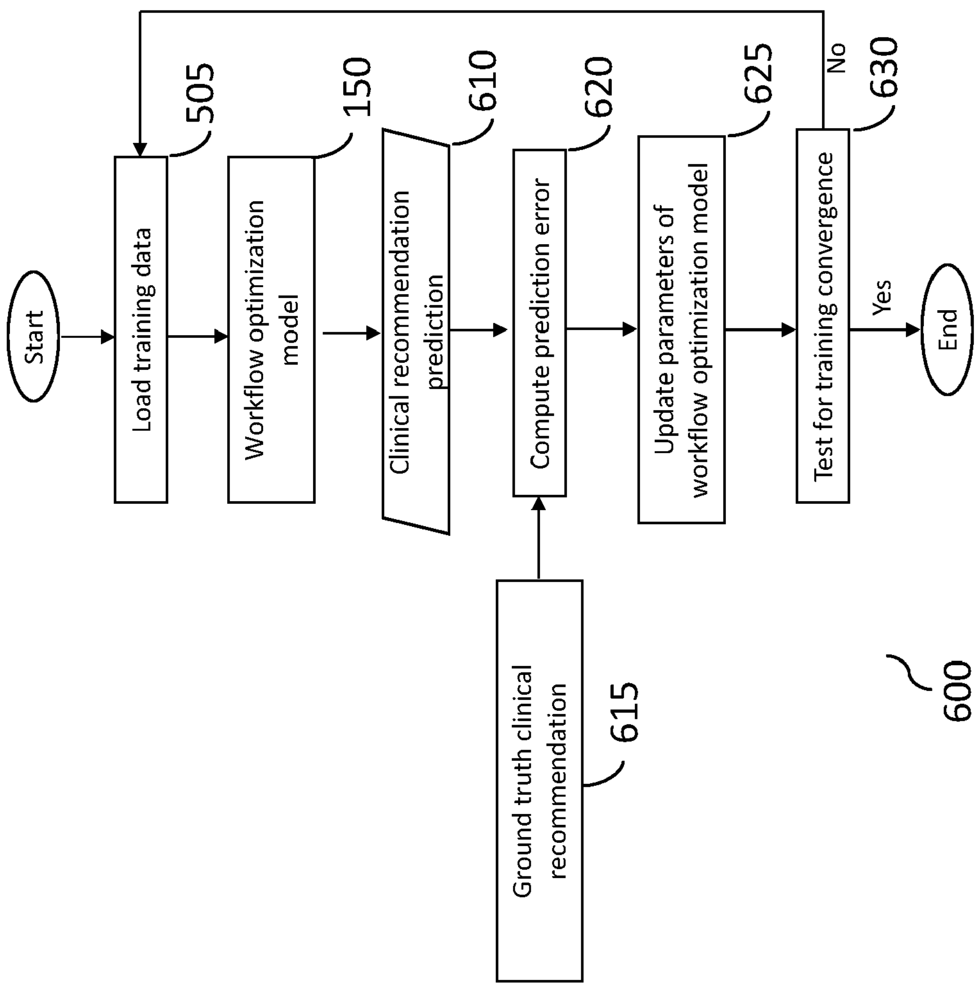

Start
Load training data
505
Workflow optimization model
150
Clinical recommendation prediction
610
Compute prediction error
620
Ground truth clinical recommendation
615
Update parameters of workflow optimization model
625
Test for training convergence
630
No
Yes
End
600


FIG. 6

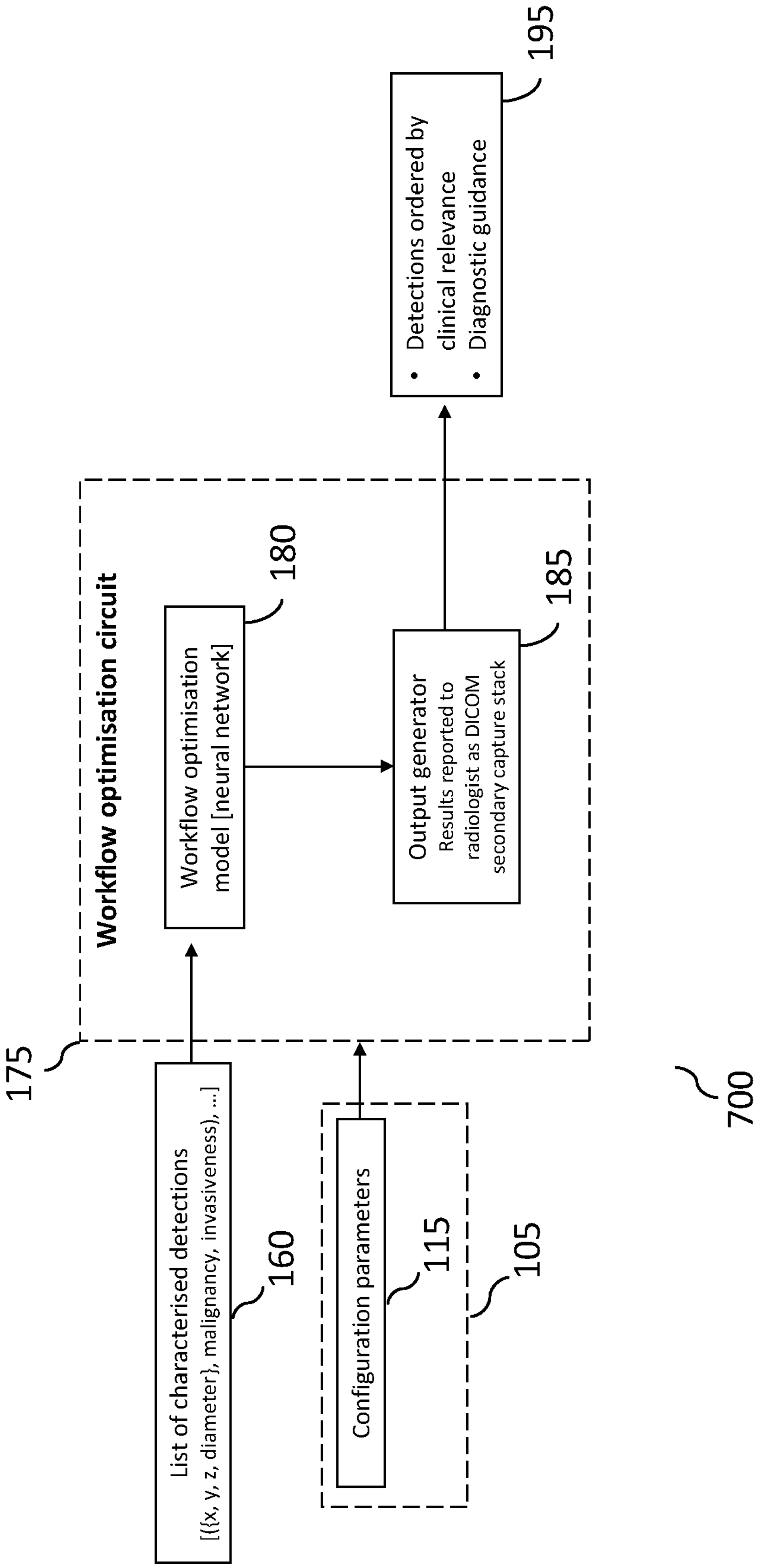
195
Detections ordered by clinical relevance
Diagnostic guidance
Workflow optimisation circuit
180
Workflow optimisation model [neural network]
185
Output generator
Results reported to radiologist as DICOM secondary capture stack
175
List of characterised detections
[({x, y, z, diameter}, malignancy, invasiveness), ...]
160
Configuration parameters
115
105
700

FIG. 7

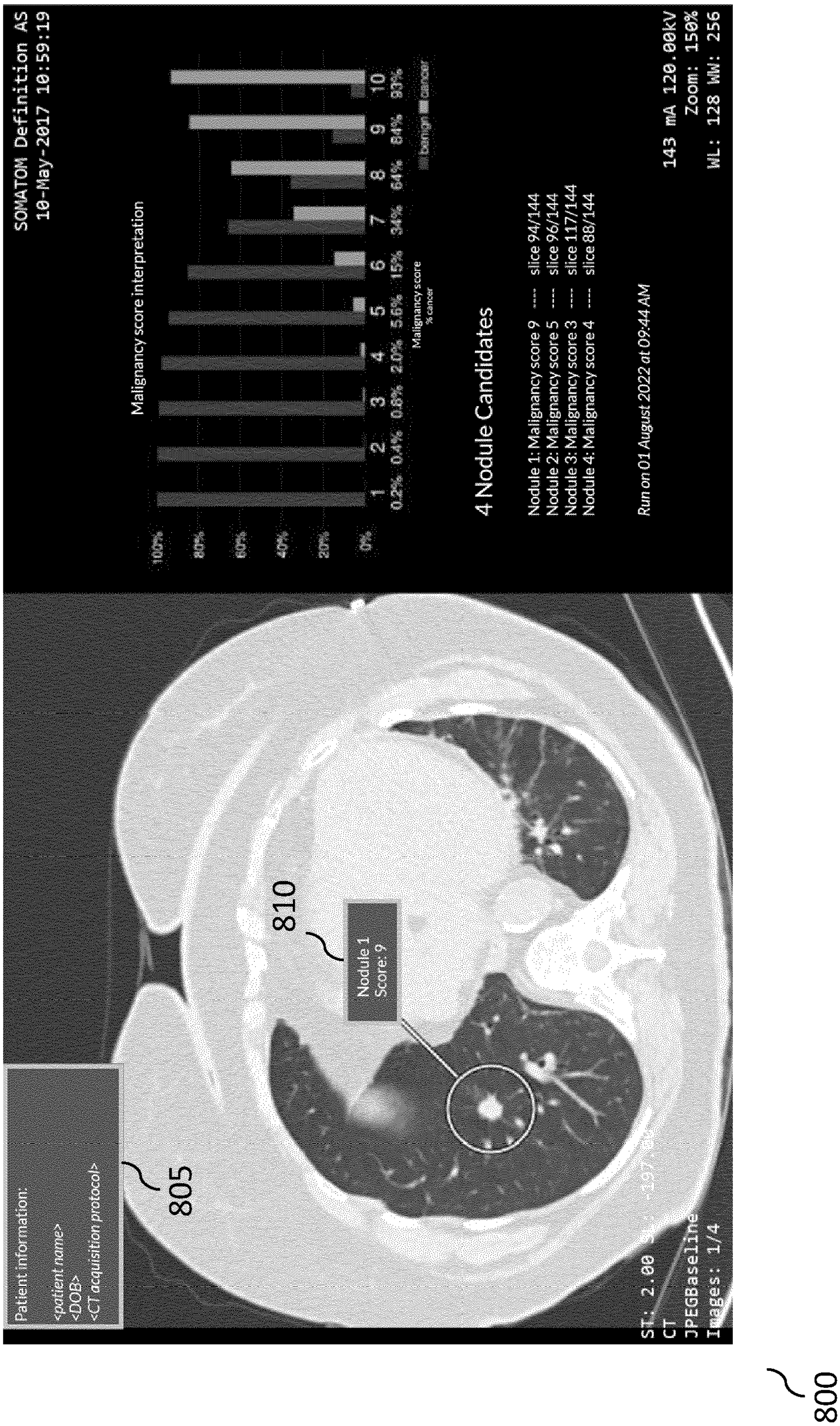
SOMATOM Definition AS
10-May-2017 10:59:19
Malignancy score interpretation
100%
80%
60%
40%
20%
0%
1 2 3 4 5 6 7 8 9 10
0.2% 0.4% 0.8% 2.0% 5.6% 15% 34% 64% 84% 93%
Malignancy score
% cancer
benign cancer
4 Nodule Candidates
Nodule 1: Malignancy score 9 ---- slice 94/144
Nodule 2: Malignancy score 5 ---- slice 96/144
Nodule 3: Malignancy score 3 ---- slice 117/144
Nodule 4: Malignancy score 4 ---- slice 88/144
Run on 01 August 2022 at 09:44 AM
143 mA 120.00kV
Zoom: 150%
WL: 128 WW: 256
Patient information:
<patient name>
<DOB>
<CT acquisition protocol>
ST: 2.00
CT
JPEGBaseline
Images: 1/4
Nodule 1
Score: 9
805
810
800

FIG. 8

Europäisches Patentamt
European Patent Office
Office européen des brevets

# EUROPEAN SEARCH REPORT

Application Number
EP 23 21 7258

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 4 020 492 A1 (SIEMENS HEALTHCARE GMBH [DE]) 29 June 2022 (2022-06-29)<br>* claims 1-2,5-8,10-13 *<br>* paragraph [0002] *<br>* paragraph [0024] *<br>* paragraph [0038] - paragraph [0039] *<br>* paragraph [0041] - paragraph [0043] *<br>* paragraph [0045] - paragraph [0046] *<br>* paragraph [0069] - paragraph [0072] *<br>----- | 1-15 | INV.<br>G16H30/20<br>G16H30/40<br>G16H50/20<br><br>ADD.<br>G06T7/00 |
| X | JP 7 187430 B2 (GENERAL ELECTRIC CO GE) 12 December 2022 (2022-12-12)<br>* claims 1-7,9-11 *<br>* paragraph [0014] - paragraph [0015] *<br>* paragraph [0026] - paragraph [0027] *<br>* paragraph [0041] - paragraph [0047] *<br>* paragraph [0053] *<br>* paragraph [0069] *<br>* paragraph [0071] *<br>* paragraph [0075] - paragraph [0078] *<br>* paragraph [0125] - paragraph [0128] *<br>----- | 1-15 | TECHNICAL FIELDS SEARCHED (IPC) |
| X | US 11 308 147 B2 (RADLOGICS INC [US] ET AL.) 19 April 2022 (2022-04-19)<br>* claim 1 *<br>* paragraph [0066] *<br>* paragraph [0068] - paragraph [0072] *<br>* paragraph [0082] *<br>* paragraph [0097] - paragraph [0100] *<br>* paragraph [0103] *<br>* paragraph [0128] - paragraph [0130] *<br>* paragraph [0138] *<br>* paragraph [0146] - paragraph [0148] *<br>----- | 1-15 | G16H<br>G06T |
| A | US 2021/225511 A1 (KIRALY ATILLA [US] ET AL) 22 July 2021 (2021-07-22)<br>* the whole document *<br>----- | 1-15 | |

The present search report has been drawn up for all claims

1

| Place of search | Date of completion of the search | Examiner |
|---|---|---|
| Munich | 25 April 2024 | Martínez Cebollada |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 23 21 7258

This annex lists the patent family members relating to the patent documents cited in the above-mentioned European search report. The members are as contained in the European Patent Office EDP file on
The European Patent Office is in no way liable for these particulars which are merely given for the purpose of information.

25-04-2024

| Patent document cited in search report | Publication date | Patent family member(s) | Publication date |
|---|---|---|---|
| EP 4020492 A1 | 29-06-2022 | NONE | |
| JP 7187430 B2 | 12-12-2022 | CN 111401398 A | 10-07-2020 |
| | | EP 3675130 A1 | 01-07-2020 |
| | | JP 7187430 B2 | 12-12-2022 |
| | | JP 2020126598 A | 20-08-2020 |
| | | US 2020211694 A1 | 02-07-2020 |
| US 11308147 B2 | 19-04-2022 | US 2014257854 A1 | 11-09-2014 |
| | | US 2022197938 A1 | 23-06-2022 |
| | | US 2022358157 A1 | 10-11-2022 |
| | | WO 2013036842 A2 | 14-03-2013 |
| US 2021225511 A1 | 22-07-2021 | CN 112292691 A | 29-01-2021 |
| | | EP 3788546 A1 | 10-03-2021 |
| | | JP 7069359 B2 | 17-05-2022 |
| | | JP 2021528751 A | 21-10-2021 |
| | | US 2021225511 A1 | 22-07-2021 |
| | | WO 2019245597 A1 | 26-12-2019 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20210225511 A1, Kiraly, A
- US 10896753 B2, Lyman


### Non-patent literature cited in the description


- **CALLISTER, M. E. J. et al.** British Thoracic Society guidelines for the investigation and management of pulmonary nodules: accredited by NICE. *Thorax,* 2015, vol. 70 (2), ii1-ii54
- **MACMAHON ; HEBER et al.** Guidelines for management of incidental pulmonary nodules detected on CT images: from the Fleischner Society 2017. *Radiology,* 2017, vol. 284 (1), 228-243
- **ARDILA, D. et al.** End-to-end lung cancer screening with three-dimensional deep learning on low-dose chest computed tomography. *Nature Medicine,* 2019, vol. 25 (6), 954-961